(19) Europäisches Patentamt

European Patent Office

Office européen des brevets

(11) **EP 1 681 015 A1**

(12) **EUROPEAN PATENT APPLICATION**

(43) Date of publication:
**19.07.2006 Bulletin 2006/29**

(51) Int Cl.:
*A61B 5/00* (2006.01)    *A61B 8/08* (2006.01)

(21) Application number: **05405026.5**

(22) Date of filing: **17.01.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Imasys SA**
**1005 Lausanne (CH)**

(72) Inventors:
• **Butz, Torsten**
  **9052 Niederteufen (CH)**

• **Thiran, Jean-Philippe**
  **1806 St-Legier (CH)**
• **Kunt, Murat**
  **1603 Grandvaux (CH)**

(74) Representative: **Ganguillet, Cyril et al**
**ABREMA**
**Agence Brevets & Marques**
**Ganguillet & Humphrey**
**Avenue du Théâtre 16**
**Case postale 5027**
**1002 Lausanne (CH)**

(54) **Temperature mapping on structural data**

(57)    Temperature imaging has been recognized to improve a variety of diagnostic and interventional procedures and to predict and prevent failures of electrical circuits and equipments. A system design and method is disclosed, wherein the thermal data is fused and mapped with the data streams coming from a US, CT, or MR scanner, giving the doctors or industrial investigators the impression of being working with a known US, CT, or MR system which has been augmented by the temperature mapping capability.

Fig. 2

**Description**

Background of the invention

Non-Destructive Evaluation (NDE) has been one of the engineering disciplines which mostly revolutionized diagnostic techniques in industry and in medicine during the last decades. MR (Magnetic Resonance), CT (Computerized Tomography), US (Ultra-Sound), and other NDE devices are being standard tools for a wide range of diagnostic fields. Furthermore they are currently changing significantly medical surgery, as their capability of visualizing intra-body structures enables surgeons to minimize the invasiveness of their interventions. Even though NDE techniques are by themselves expensive, they are potentially even interesting from a financial point of view, as they can significantly decrease the expensive hospitalization time of patients. Similar arguments apply to industrial NDE which was able to bring quality assurance and failure prediction to an impressive performance.

Information coming from one NDE device is always restricted to a particular characteristic of the intra-body physics. For example, the information that can be drawn from MR devices or CT scanners is related but still very different. While medical MR devices image the soft tissues, CT scanners are unable to differ between different soft tissues while giving information about the skeleton structure of the patient. In practice, both imaging systems are therefore used when necessary, in order to determine the complementary information from both devices. The result is multimodal information, i.e. information which can practically not be provided by one single system.

As used herein, "multimodal" means use of at least two imaging modes which differ by the physical characteristics of the scene they image during the data acquisition process.

One of the research directions consists of determining new imaging modalities which are able to measure intra-body characteristics. One of these characteristics is the intra-body temperature of industrial specimen or patients. Temperature is a highly discriminative characteristic for e.g. cancer tissues or design errors in electrical circuits. Several approaches to temperature imaging have been proposed, but just a few have been able to reach any real market value.

Devices enabling data acquisition of thermal maps comprise MR scanners (intra-body), infrared devices (surface), and passive microwave imaging systems (intra-body). Thermal maps are considered to give pertinent information. In the case of medical imaging, the temperature maps acquired by the imaging device can give information about the intra-body temperature distribution during thermal ablation, or about the presence, respectively absence, of cancerous tissues in human bodies. In the context of industrial applications, thermal maps are particularly valuable for the prediction and prevention of electrical equipment circuit failures.

But temperature by itself has just a restricted practical value if it cannot be combined with other imaging modalities such as US, CT, or MR data, in order to not only give intra-body temperature maps, but also to relate them to the underlying physical structure of the patient or specimen under study. For example, increased intra-body temperature is not sufficient to conclude on the presence of cancerous tissue due to the inhomogeneity of biological tissue. It could also come from a local inflammation of the biological tissue. Underlying anatomical data reflecting this inhomogeneity can therefore increase the diagnostic quality of the acquired temperature maps significantly.

Thermal maps are medically and industrially most useful in conjunction with anatomical or structural images in order to provide the medical doctors and investigators with information complementary to the thermal data. In the case of cancer detection, the anatomical images acquired by e.g. US or CT devices ensure that not only cancerous tissues are detected, but also its exact anatomical location can be determined, enabling the medical doctors to optimize their treatment plans. In the context of electrical circuits, the temperature maps get expressive when being related to the underlying circuit design.

Presently, different imaging modalities, such as thermal maps and anatomical information, could only be provided sequentially, but not simultaneously. As a result, the following disadvantages of previous approaches to thermal imaging exist:

First, anatomical and structural information is a fundamental prerequisite for medical doctors and investigators during medical diagnosis or industrial quality testing in order to locate sensitive anatomical structures, e.g. during thermal ablation, or to relate failure sensitive regions of an integrated circuit to the underlying circuit design which can be improved thereafter. Therefore, having access to mere temperature maps without having simultaneously the related structural information is a highly limiting factor, as temperature maps by themselves do not provide any information about the underlying structure.

Second, in the case of medical applications, the medical doctors are used to inspect anatomical data, but do not have much practical experience in interpreting thermal maps due to the novelty of thermal imaging devices. Therefore an appropriate combination of thermal maps with conventional anatomical imaging modalities would heavily facilitate the medical doctors' tasks of interpreting the acquired thermal maps.

Third, if thermal and structural information are acquired in a sequential manner, it is required to physically fix the patient, or the investigated industrial part during and between the successive imaging processes and to determine exactly the position of the imaging devices in order to be able to fuse and visualize the multimodal data sets without mutual displacements.

Fourth, even if both data from the same body portion could be acquired simultaneously by two different imaging devices, a pure superimposition of the resulting images would not be optimal, as redundant or non relevant information would get displayed as well.

Summary of the Invention

The present invention solves the problems described above by providing means to optimally fuse and visualize the data coming from individual imaging devices.

An object of the invention is a virtual multimodal imaging device comprising :

- a first monomodal imaging subsystem with first sensing means and with first processing means, providing digitized thermal map data from a sensed body,

- a second monomodal imaging subsystem with second sensing means and with second processing means, providing digitized structural map data from said sensed body, and

- a multimodal signal processor performing point-to-point registration of thermal and structural maps, data fusion into a multimodal data set and visualisation of said multimodal data set.

Thus, the processing system which receives the data streams from the respective imaging devices performs signal conditioning, image registration, and data fusion, in order to provide the information from the plurality of input data streams in a virtually fused single data stream.

Preferably, said processor extracts corresponding image features of said thermal map and of said structural map for the registration process. The resulting digital data stream guarantees a point-to-point correspondence of the input data streams from the different imaging devices.

Preferably, said processor extracts complementary image features of said thermal map and of said structural map for the data fusion step. Thereby, the system removes redundant information from the different data streams in order to guarantee the visualization of both a complete and very compact representation of all the information coming from the different input data streams.

Preferably, the thermal data of said multimodal data set are color coded and overlaid to the structural data. The system visualizes the resulting single data stream on a computer screen.

Preferably, configuration information is streamed back from said processing system to said imaging subsystems.

The system has several inputs for the different imaging devices, one of which provides the thermal data, the other (s) the complementary structural information. Data streams from the thermal imaging device and one, or more than one, structural imaging device are streamed over standard networking and data streaming connections, such as USB2, IEEE1394, Ethernet, S-Video, or others, towards the processing system. In the case of analogue streaming connections, such as S-Video, a state-of-the-art analogue-to-digital converter or frame-grabber digitizes the analogue data before passing the resulting digital representation to the processor.

In order to bring the data into point-to-point correspondence, the imaging devices can be linked mechanically and calibrated to pre-determine the transformation which brings the data into correspondence. The resulting transformation can thereafter be applied in real-time. As an alternative, a software tool performs this task by maximizing the mutual information between image features with respect to possible image rotations and displacements. Further, the software will extract the most complementary and pertinent image features and characteristics of the different image modalities before fusing the resulting data into one multi-modal data set. As a final step, the resulting image representing the multimodal information is being visualized on a computer screen.

As used herein "real-time" capability of the final system refers to a real-time capability which does not significantly differ from the real-time capabilities of the individual imaging subsystems which are connected to the core device. The system streams two data streams to the signal processor, e.g. the 3D thermal data from an MR device, noted $T(x,y,z)$, and the 2D structural data from a US device, noted $S(x',y')$, where $x, y,$ and $z,$ resp. $x'$ and $y',$ parameterize the discrete imaging space of the thermal MR data T, resp. the structural US data S. A mapping $m_\alpha: (x',y') -> (x,y,z)$ provides a point-to-point correspondence between both, the imaging space of T and the imaging space of S, with $\alpha$ being the registration parameters for rigid or non-rigid registration, i.e. $\alpha$ parameterizes translations and rotations of the 2D US data frames within 3D (as the MR data is 3D). The mapping parameters $\alpha$ and image features which allow the determination of $\alpha$ have to be determined simultaneously. Mathematically, the feature extraction can be formalized as an image mapping, $k_\beta$: $T(x,y,z)$->$kp(T(x,y,z))$, resp. $ly$: $S(x',y')$->$l_\gamma(S(x',y'))$, with $\beta$, resp. $\gamma$, being the feature extraction parameters. The aim of using image features instead of the raw image data for the registration process reflects the fact that not all information contained in multimodal data is pertinent for the registration process. Some image characteristics solely present in one of the modalities, such as imaging noise, cannot give any reliable input to the registration process, but rather decrease reliability of the algorithm. Therefore the feature extraction block within the multimodal registration process detects the image features most pertinent for the point-to-point correspondence of the input images and removes those not being of any use for this aim.

The optimization objective which allows simultaneous extraction of the most related image features and the determination of the optimal registration parameters $\alpha$ is written

$$e(F_T, F_S) = (I(F_T, F_S)/H(F_S, F_T)),$$

where $I(.,.)$ stands for mutual information, $H(.,.)$ for joint entropy, $F_T$ is a random variable with a probability density estimated from the thermal data T, and $F_S$ is a random variable with a probability density estimated from the structural data S.

Therefore the resulting data registration process can be formalized as

$$(\alpha^{opt}, \beta^{opt}, \gamma^{opt}) = \max_{\alpha, \beta, \gamma} e(F_{k\beta(T(m\alpha(x,y,z)))}, F_{l\gamma(S(x'y'))}).$$

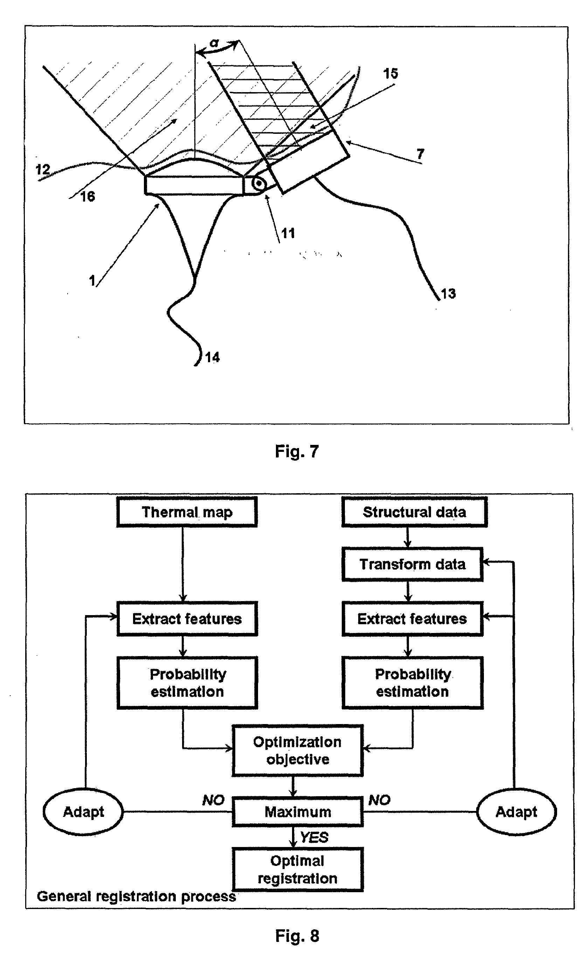

This process is outlined in fig. 8. The action of adapting registration parameters and image features refers to adapting the parameters $\alpha$, $\beta$, and $\gamma$. For maximization, any adequate optimization algorithm can be used.

In contrast to the first data feature extraction related to image registration as described in the previous paragraph, the second feature extraction for data fusion aims to extract the features of the initial datasets which are most complementary to each other while removing redundant information from the datasets. Mathematically, the feature extraction process is written the same way as in the previous paragraph, even though its implementation might differ. Therefore, the feature extraction from the input thermal and structural maps is represented by a mapping $o_\delta$: $T(m_\alpha(x,y,z))$ ->$o_\delta(T(m_\alpha(x,y,z)))$, resp. $u_\varepsilon$: $S(x',y')$->$u_\varepsilon(S(x',y'))$. $\delta$, resp. $\varepsilon$, represent again a particular scale of the scale space decomposition of the initial MR thermal maps, $T(x,y,z)$, resp. the structural data of the US device, $S(x',y')$. The extraction process is driven by the minimization of the same optimization function e as in the previous paragraph. Mathematically this can be written

$$(\delta^{opt}, \varepsilon^{opt}) = \min_{\delta, \varepsilon} e(F_{o\delta(T(m(x,y,z)))}, F_{u\varepsilon(S(x'y'))}).$$

For the optimization process, again any adapted algorithm can be employed.

The fact that while for registration the optimization objective e has to be maximized, the optimization objective has to be minimized for data fusion, reflects the fact that data fusion aims to keep all available information of the input data and to remove the redundant information. In the case of registration, it is just this redundant information, i.e. the information that is present in both input datasets, that is able to drive the registration process towards the optimal point-to-point correspondence.

The resulting datasets, $o_\delta(T(m_\alpha(x,y,z)))$ and $u_\varepsilon(S(x',y'))$, are fused thereafter. The final data fusion is a fundamental step with respect to the general design of the system, as it is thanks to the fusion result that the medical doctor or industrial investigator has the impression of being working with only one single physical system. For this aim, the thermal map data, $o_\delta(T(m_\alpha(x,y,z)))$, resulting from the previously described signal processing steps, are getting color coded.

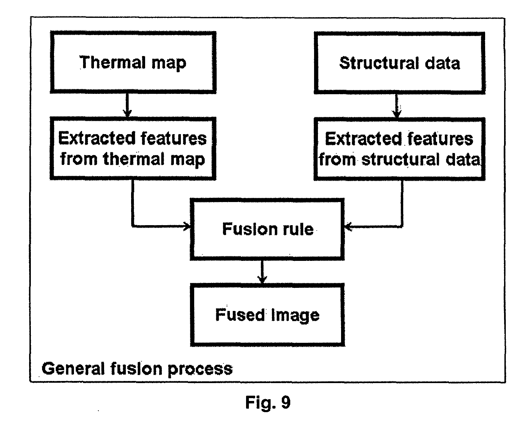

The resulting color mapped thermal data can be overlaid on the structural data, $u_\varepsilon(S(x'y'))$, resulting in a virtually augmented image. This process is the implementation of the so called "fusion rule" of fig. 9. It can be programmed by commercially available visualization software packages, like OpenGL or DirectX. These softwares can also be employed for the real-time visualization of the resulting multi-modal data stream.

Further features and advantages of the invention will appear to those skilled in the art by means of the following description of a particular embodiment in connection with the drawings.

Brief Description of the drawings

Fig. 1 shows a block diagram of the system design.

Fig. 2 is a block diagram showing in more details the processing system of fig. 1.

Fig. 3 shows an illustrative implementation of the system design of fig. 1. Thermal imaging is performed by a microwave (MW) device; meanwhile an ultrasound (US) device provides the anatomical information.

Fig. 4 presents the US subsystem of fig. 3.

Fig. 5 presents the MW subsystem of fig. 3.

Fig. 6 presents in more detail The MW subsystem of fig. 5. In particular the multi-frequency spiral antennas are shown, which build up the MW transducer.

Fig. 7 shows how the US and MW transducers are connected.

Fig. 8 is a block diagram of the image registration process.

Fig. 9 is a block diagram of the image fusion process. Thermal map and structural data refer to the already registered data.

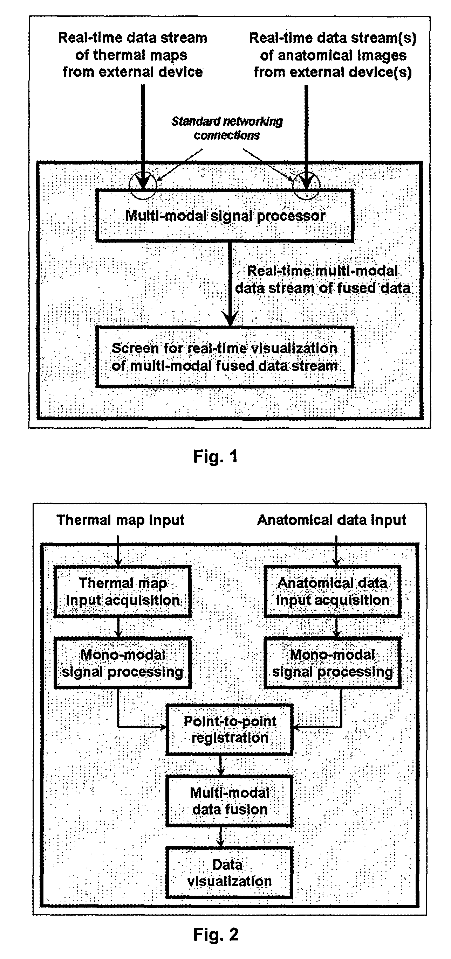

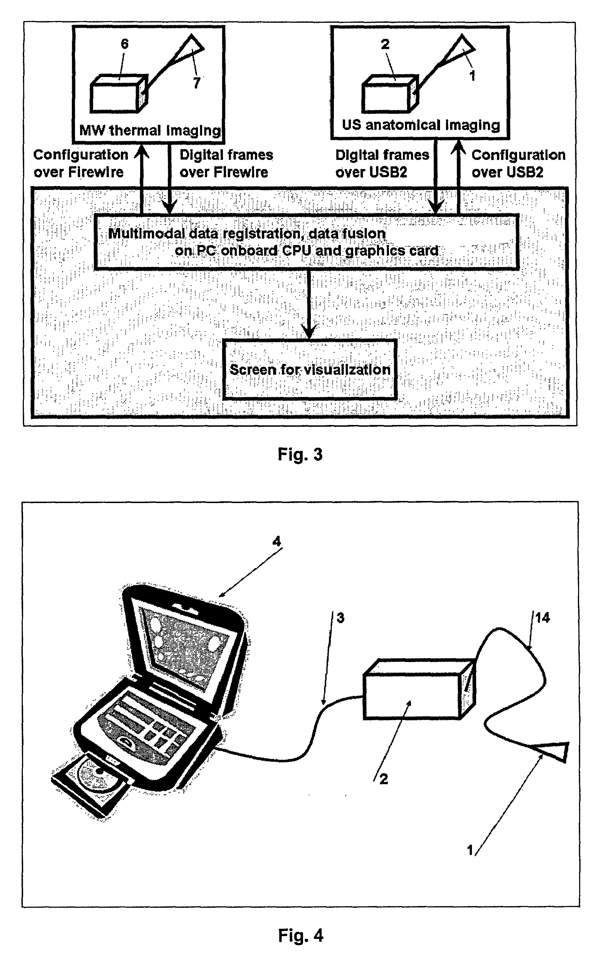

Fig. 1 presents the general design of the system. The data from the different imaging devices are streamed over standard networking or data streaming connection like USB2, IEEE1394, Ethernet to the processing system. As standard networking connections are being employed, the implementation of the frame capturing capability of the system is related to the technical specifications of the subsystem or the frame-grabber provider. Necessary device drivers and applications interfaces (APIs) are provided from commercial suppliers.
If the imaging subsystems have been designed in such a way, configuration information is getting streamed back from the processing system to the imaging subsystems in order to provide the investigator with the impression of being interacting directly with the subsystems. The data from the input devices are getting processed and fused inside the processing system before getting visualized on a computer screen.
Performing the different image processing steps shown in fig. 2 continuously as the data arrives over the input networking connections, results in a virtual multi-modal imaging device. The medical doctor or industrial investigator can therefore interpret the acquired multimodal datasets simultaneously and, if the subsystems have been designed in that way, interact with the different subsystems, i.e. the MR and US devices, as if they were just one single multi-modal device which generates just one single data stream.
Fig. 2 shows roughly the different image processing steps that are performed by the processing system before on-screen visualization. In more detail, the processing system performs data registration in order to guarantee point-to-point correspondence of the input data streams, data feature extraction to provide the most pertinent and only pertinent information to the investigator, and data fusion to give the investigator the impression of being working with only one single imaging device. In the following example, these individual digital image processing steps are described in detail for a specific system implementation.

Example: Implementation for Liver Tumor Thermal Ablation

In fig. 3, a specific system setup for the monitoring of thermal ablation of liver tumors is lined out. Liver is one of the major tumor sites in the western world with an incidence of 680'000 cases/year. During thermal ablation of liver cancers, thin needles are introduced percutaneously until their tips reach inside the tumor volume. At the tips, radiofrequency heats the tumor tissue up to a temperature guaranteeing cell death. In this procedure, two characteristics are fundamental for the success of the treatment: On the one hand, all the cancerous tissue has to be killed, and on the other hand, as few healthy tissue as possible should be ablated. For this, real time monitoring of anatomy, needle positions, and temperature profiles should be provided, which is done with the specific system implementation presented herein.
There are two imaging subsystems connected to the processing system implemented on a state-of-the-art personal computer (PC): On the one hand, a microwave (MW) imaging device which provides 2D intra-body temperature maps of the patient, and on the other hand a ultrasound (US) imaging system which images 2D slices of the patient's anatomy and the needle positions. As schematically shown in figs 3, 4 and 5, both systems are connected to the PC through real-time networking connections through which the anatomical data and temperature maps are streamed in real-time. For this, the US device uses a USB2 connection, while the MW system employs a Firewire connection.

The Personal Computer (PC) 4 is provided with following components:

- US device drivers and C++-APIs for USB2 communication from Telemed (see ref. [1]);
- Firewire drivers and C-APIs from Unibrain (see ref. [6]);
- Software for multimodal data registration,
- VTK for data fusion and visualization (see ref. [12]);
- Qt for graphical user Interface (see ref. [13]).

Using commercially available drivers and C/C++ application interfaces (APIs), the specifications of which may be found in ref. [1] and ref. [6], the processing system receives in real-time the respective datasets from the connected subsystems. The APIs are called from individual C-threads on the PC 4 which are implemented in order to receive the data from the individual imaging subsystems individually, but in a synchronized fashion.

The employed US subsystem is the commercially available Echoblaster 128 produced by Telemed, Lithonia (see ref. [1]). The two dimensional US frames are streamed over the USB2 connection 3 from the US beamformer 2 to the PC 4, and changes in configuration are streamed back from the PC 4 to the beamformer 2. Furthermore, Telemed provides the hardware drivers and C++ application interface (API) for Windows, based on the DirectX technology from Microsoft. A variety of US transducers 1 is also proposed by Telemed, enabling any programmer to easily implement a fully functional PC based US device.

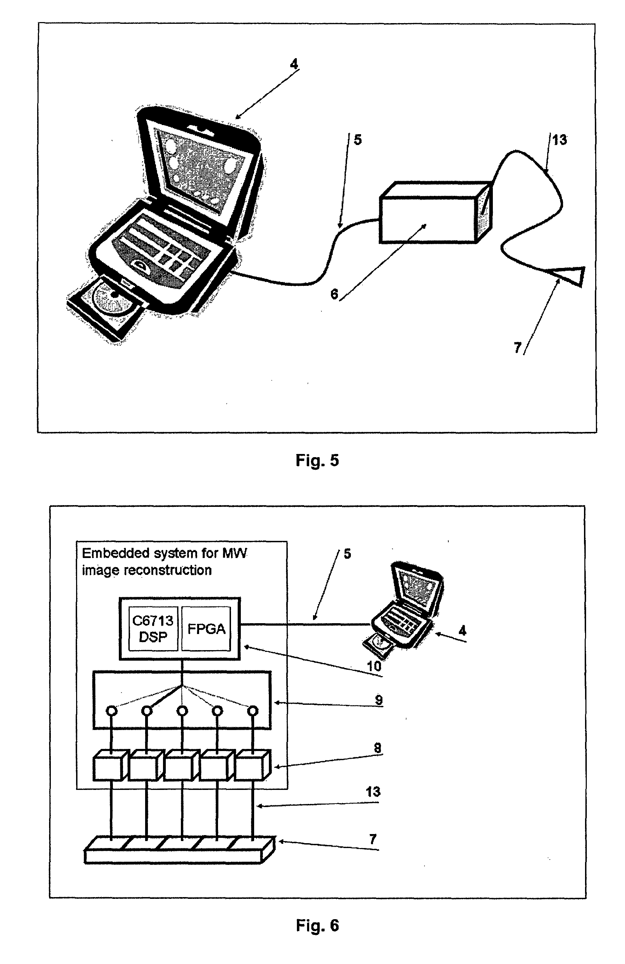

Fig. 6 depicts the hardware components of the MW imaging subsystem. The constructed microwave subsystem comprises an array 7 of multi-frequency spiral MW antennas as disclosed by Jacobsen and Stauffer in ref. [2]. The signal sensed by the individual antennas passes through individual Dicke null-balancing radiometers 8, as described by Jacobsen and al. in ref. [3]. The analogue signal from each radiometer 8 is directly related to the brightness temperatures at the locations sensed at different frequencies. An embedded analogue-to-digital converter 9 from Orsys, such as the ORS-116 (see ref. [5]), converts these analogue brightness temperatures into their digital representations. As the analogue-to-digital converter 9 has several analogue inputs, the brightness temperatures from the different antennas can be digitized consecutively. The result is a two dimensional grid of brightness temperatures, where the number of grid points are determined by the number of frequencies that can be sensed by the individual antennas, and the number of antennas that are connected in the antenna array 7.

The brightness temperatures are directly related to the real intra-body temperatures at the different locations. In order to reconstruct a two dimensional grid of real intra-body temperatures from the brightness temperatures, the algorithm disclosed by Jacobsen and Stauffer in ref. [4] is getting applied to the output grids from the Orsys analogue-to-digital converter 9. In fact, as the algorithm of ref. [4] reconstructs 1D temperature profiles, it is applied consecutively to the brightness temperatures of the individual antennas in the antenna array 7. The combination of the reconstructed 1D temperature profiles results in a 2D temperature map. The reconstruction algorithm from ref. [4] is implemented on the embedded Compact C6713 system 10, sold by Orsys (see ref. [5]). The analogue-to-digital converter 9 from Orsys can actually be plugged directly on the microbus of the Compact C6713 embedded system 10. Consecutive frames of 2D temperature maps are streamed over the firewire connection 5 of the Compact C6713 system to the firewire connector of the PC 4, while system configuration parameters are streamed back from the PC 4 to the Compact C6713 embedded device. On the PC 4, the firewire drivers and C-APIs from Unibrain (see ref. [6]) provide the programmer with an easy to use tool to implement a completely functional temperature monitoring imaging device.

In order to overlay the temperature maps from the MW device on the US frames with a guaranteed point-to-point correspondence, the US transducer 1 is physically linked to the MW antenna array 7. This guarantees that both datasets are acquired within the same imaging plane and that the imaged regions of both devices overlap significantly. The link between the two transducers is outlined in fig. 7. The two transducers are connected through a rotational joint 11, which allows adapting the rotational angle $\alpha$ according to the local patient's anatomy, e.g. curved or straight skin surface 12. As a result, the two datasets lay both in the same imaging plane, but are mutually rotated by an angle $\alpha$. The field of view of the MW antenna array 7 is indicated by 15 and the field of view of the US transducer 1 is indicated by 16. The analogue connection between US transducer 1 and beamformer 2 is indicated by 14 and analogue connection between antenna array 7 and system 6 is indicated by 13. Therefore, before a data overlay with point-to-point correspondence can be provided, this angle $\alpha$ has to be determined in order to compensate for the rotational difference. This process of bringing the two datasets into spatial correspondence provides data registration. The specific implementation of the registration algorithm is implemented on the PC 4.

The registration process which enables to compensate for the rotational offset of $\alpha$ between the US and MW scans is outlined in fig. 8. The system streams via connections 5 and 3 on the one hand the thermal data from the MW device 6, noted T(x,y), and the structural data from the US device 2, noted S(x',y') to the signal processing PC 4. x and y, resp. x' and y', parameterize the discrete imaging space of the thermal MW data T, resp. the structural US data S, as described in the previous paragraphs. As depicted in fig. 7, the imaging space of the MW data and the imaging space of the US frames differ by a rotational angle $\alpha$. In order to find continuously a point-to-point correspondence between both, the varying angle $\alpha$ shall be continuously determined in order to compensate for it. This corresponds to determining the mapping $m_\alpha$: (x',y')->(x,y), with $\alpha$ being the single valued rotational transformation of the mapping. The datasets S and T represent different information about the investigated patient, and the determination of this mapping is described in the following.

In fact both, the rotational angle $\alpha$ and image features which allow the determination of $\alpha$ have to be determined simultaneously. The feature extraction step can have a variety of specific implementations, e.g. considering a prior information about the features to be extracted, discretely or continuously parameterized features, etc. With respect to the specific implementation described in this example, the feature extraction parameters $\beta$ and $\gamma$ represent a particular scale of the scale space image decomposition described by Lindenberg in ref. [7]. The feature extraction block within the multimodal registration process detects the image features most pertinent for the point-to-point correspondence of the input images and removes those not being of any use. The fact that in this particular implementation the imaging features are restricted to a specific scale of the scale space decomposition of the initial datasets reflects a known prior information about which image features will result in good registration. Still, the exact scale is not being fixed from the beginning, as the best scale in the scale space decomposition is not guaranteed to remain constant. Rather, it might change with changing parameters in the image acquisition process, such as e.g. a changing frequency used for the US image acquisition, since Telemed provides multi-frequency US transducers.

The optimization objective which allows simultaneous extraction of the best scale of the scale space image decomposition and the determination of the optimal registration angle $\alpha$ is written

$$e(FT,FS) = (I(FT,FS)/H(FS,FT))$$

as indicated above. FT is estimated from the thermal data T, and FS is estimated from the structural data S, according to the teaching of Thomas A. Cover in ref. [8]. Histogramming is being employed as the probability estimator for FT and FS, as described by T. Butz in ref. [9].

The action of adapting registration parameters and image features refers to adapting the parameters $\alpha$, $\beta$, and $\gamma$. For maximization, an optimization algorithm such as Powell (see ref. [10]) or genetic optimization (see ref. [11]) can be used.

In contrast to the data feature extraction related to image registration as described in the previous paragraph, the feature extraction for data fusion aims to extract the features of the initial datasets which are most complementary to each other while removing redundant information from the datasets. The feature extraction from the input thermal maps, resp. the US data, is represented by a mapping $o_\delta$: $T(m_\alpha(x,y))-->o_\delta(T(m_\alpha(x,y)))$, resp. $u_\epsilon$: $S(x',y')->u_\epsilon(S(x',y'))$. $\delta$, resp. $\epsilon$, represent again a particular scale of the scale space decomposition accordin to ref. [7] of the initial MW thermal maps, $T(x,y)$, resp. the anatomical data of the US device, $S(x',y')$. The extraction process is driven by the minimization of the same optimization functional e as in the previous paragraph.

For the optimization process again, an algorithm, such as Powell or genetic optimization, can be employed. The resulting datasets, $o_\delta(T(m_\alpha(x,y)))$ and $u_\epsilon(S(x',y'))$, will be fused thereafter.

For the data fusion, the thermal maps, $o_\delta(T(m_\alpha(x,y)))$, resulting from the previously described signal processing steps, are color coded so as to reflect a natural interpretation, e.g. hot spots being red and cold spots being blue. When a thermal data, $t(x,y)=o_\delta(T(m_\alpha(x,y))$, is initially represented by a scalar within $[t_{min},t_{max}]$, such a thermal mapping is being performed by the following color coding equation:

$$\begin{pmatrix} r \\ g \\ b \end{pmatrix}(x,y) = \begin{pmatrix} 0 \\ 2 \cdot t(x,y)/t_{max} \\ 1 - 2 \cdot t(x,y)/t_{max} \end{pmatrix}, t \in [t_{min}, t_{max}/2],$$

$$\begin{pmatrix} r \\ g \\ b \end{pmatrix}(x,y) = \begin{pmatrix} 2 \cdot t(x,y)/t_{max} \\ 1 - 2 \cdot t(x,y)/t_{max} \\ 0 \end{pmatrix}, t \in [t_{max}/2, t_{max}].$$

The resulting color mapped thermal data can be semi-transparently overlaid on the structural data, $u_\varepsilon(S(x',y'))$, resulting in a virtually augmented image. In this example, the software VTK (Visualization Tool Kit) (see ref. [12]) is employed as the data fusion and visualization package.

The software Qt available from Trolltech (see ref. [13]) is being employed for the implementation of the graphical user interface, which is designed in a way that the doctor can interact directly over the USB2, resp. Firewire, connection to the US device, resp. MW device, with the imaging subsystems. Thus, performing the described different image processing steps continuously as the data arrive over the input networking connections, results in a virtual multi-modal imaging device. The doctor can therefore interpret the acquired multimodal datasets simultaneously and interact with the MW and US subsystems, as if they were just one single multi-modal device which generates just one single data stream.

The invention as described herein above is capable to provide the industrial investigators and medical doctors simultaneously and in real-time, or off-line, with both structural data on the one hand and thermal maps on the other hand. Furthermore, as the information from different imaging modalities is combined by the multimodal signal processor, also the pertinent and complementary information from the different modalities is combined, resulting in a virtually augmented single system with increased value versus the individual subsystems.

The open standard technology of the data stream connections between the individual data acquisition systems and the processor enables removal and addition of imaging subsystems according to need and comfort of the industrial investigators or medical doctors. This applies to 1D, 2D, 3D or mixed data and data sequences.

References

[1] http://www.telemed.lt

[2] S. Jacobsen and P.R. Stauffer Multifrequency Radiometric Determination of Temperature Profiles in a Lossy Homogeneous Phantom Using a Dual-Mode Antenna with Integral Water Bolus IEEE Transactions on Microwave Theory and Techniques, vol. 50, no. 7, pp. 1737-1746, July 2002.

[3] S. Jacobsen, P.R. Stauffer, and D. Neuman Dual-mode Antenna Design for Microwave Heating and Noninvasive Thermometry of Superficial Tissue Disease IEEE Transactions on Biomedical Engineering, vol. 47, pp. 1500-1509, November 2000.

[4] S. Jacobsen and P.R. Stauffer Nonparametric 1-D Temperature Restoration in Lossy Media Using Tikhonov Regularization on Sparse Radiometry Data IEEE Transactions on Biomedical Engineering, vol. 50, no. 2, pp. 178-188, February 2003.

[5] http://www.orsys.de

[6] http://www.unibrain.com

[7] T. Lindeberg Scale-space theory: A basic tool for analyzing structures at different scales J. of Applied Statistics, vol. 21(2), pp. 224-270, 1994

[8] Thomas A. Cover Elements of Information Theory Wiley-Interscience

[9] T. Butz From Error Probability to Information Theoretic Signal and Image Processing Thèse No. 2798, ITS, EPFL

[10] William H. Press Numerical Recipes in C Cambridge University Press

[11] http://lancet.mit.edu/ga/

[12] http://www.vtk.org

[13] http://www.trolltech.com

**Claims**

1.  A virtual multimodal imaging device comprising :

    - a first monomodal imaging subsystem with first sensing means and with first processing means, providing digitized thermal map data from a sensed body,
    - a second monomodal imaging subsystem with second sensing means and with second processing means, providing digitized structural map data from said sensed body, and
    - a multimodal signal processor (4) performing point-to-point registration of thermal and structural maps, data fusion into a multimodal data set and visualisation of said multimodal data set.

2.  A device as claimed in claim 1, wherein said processor (4) extracts corresponding image features of said thermal map and of said structural map for the registration process.

3.  A device as claimed in claim 2, wherein said processor (4) extracts complementary image features of said thermal map and of said structural map for the data fusion step.

4.  A device as claimed in anyone of claims 1 to 3, wherein the thermal data of said multimodal data set are color coded and overlaid to the structural data.

5.  A device as claimed in anyone of the preceding claims, wherein configuration information is streamed back from said processing system to said imaging subsystems.

6.  A device as claimed in anyone of the preceding claims, wherein said first imaging subsystem comprises a microwave (MW) imaging device (6, 7).

7.  A device as claimed in claim 6, wherein said microwave imaging device (6, 7) comprises an array (7) of multi-frequency MW antennas associated to Dicke null-balancing radiometers (8) and an analogue-to-digital converter (9).

8.  The device as claimed in claim 7, wherein said microwave imaging device is connected to said processor (4) by a firewire connection (5).

9.  A device as claimed in any one of the preceding claims, wherein said second imaging subsystem is a ultrasound subsystem (1, 2).

10. A device as claimed in claim 9, wherein said ultrasound subsystem (1, 2) is connected to said processor (4) by a USB2 connection (3).

Real-time data stream
of thermal maps
from external device

Real-time data stream(s)
of anatomical images
from external device(s)

*Standard networking
connections*

Multi-modal signal processor

Real-time multi-modal
data stream of fused data

Screen for real-time visualization
of multi-modal fused data stream

**Fig. 1**

Thermal map input

Anatomical data input

Thermal map
input acquisition

Anatomical data
input acquisition

Mono-modal
signal processing

Mono-modal
signal processing

Point-to-point
registration

Multi-modal
data fusion

Data
visualization

**Fig. 2**

**Fig. 3**

**Fig. 4**

**Fig. 5**

Embedded system for MW image reconstruction

C6713 DSP | FPGA

**Fig. 6**

**Fig. 7**

General registration process

**Fig. 8**

EP 1 681 015 A1

**Thermal map**

**Structural data**

**Extracted features from thermal map**

**Extracted features from structural data**

**Fusion rule**

**Fused Image**

General fusion process

**Fig. 9**

**European Patent Office**

**EUROPEAN SEARCH REPORT**

Application Number

EP 05 40 5026

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | US 2003/028114 A1 (CASSCELLS S. WARD ET AL) 6 February 2003 (2003-02-06)<br>* page 9, left-hand column, line 7 - line 10 *<br>* page 10, right-hand column, line 42 - line 47 *<br>* paragraphs [0039], [0042], [0152], [0200]; figures 1-36 * | 1-6,8-10 | A61B5/00<br>A61B8/08 |
| Y | WIRTH M A ET AL: "Point-to-point registration of non-rigid medical images using local elastic transformation methods"<br>IMAGE PROCESSING AND ITS APPLICATIONS, 1997., SIXTH INTERNATIONAL CONFERENCE ON DUBLIN, IRELAND 14-17 JULY 1997, LONDON, UK,IEE, UK,<br>vol. 2, 14 July 1997 (1997-07-14), pages 780-784, XP006508401<br>ISBN: 0-85296-692-X<br>* page 781, left-hand column, line 33 - right-hand column, line 20 *<br>* page 780, left-hand column, line 22 - line 44 * | 1-6,8-10 | |
| A | PATENT ABSTRACTS OF JAPAN<br>vol. 015, no. 402 (P-1262),<br>14 October 1991 (1991-10-14)<br>& JP 03 162637 A (OLYMPUS OPTICAL CO LTD),<br>12 July 1991 (1991-07-12)<br>* abstract * | 7 | TECHNICAL FIELDS SEARCHED (Int.Cl.7)<br>A61B |
| A | EP 1 267 736 A (N.V. THERMOCORE MEDICAL SYSTEMS S.A; THERMOCORE MEDICAL SYSTEMS NV) 2 January 2003 (2003-01-02)<br>* paragraphs [0089], [0090]; figures 1-10 * | 1 | |

-/--

The present search report has been drawn up for all claims

| Place of search | Date of completion of the search | Examiner |
|---|---|---|
| The Hague | 28 June 2005 | Hunt, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 05 40 5026

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 6 640 130 B1 (FREEMAN JENNY E ET AL) 28 October 2003 (2003-10-28) * column 8, line 12 - line 29; figure 1 * ----- | 1 | |
| A | US 2003/011622 A1 (YOMDIN YOSEF ET AL) 16 January 2003 (2003-01-16) * paragraph [0081]; figures 1-34 * ----- | 1 | |

TECHNICAL FIELDS
SEARCHED     (Int.Cl.7)

The present search report has been drawn up for all claims

| Place of search | Date of completion of the search | Examiner |
|---|---|---|
| The Hague | 28 June 2005 | Hunt, B |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.                     EP 05 40 5026

This annex lists the patent family members relating to the patent documents cited in  the above-mentioned European search report.
The members are as contained in the European Patent Office EDP file on
The European Patent Office is in no way liable for these particulars which are merely  given for the purpose of information.

28-06-2005

| Patent document cited in search report | | Publication date | Patent family member(s) | | Publication date |
|---|---|---|---|---|---|
| US 2003028114 | A1 | 06-02-2003 | US | 6615071 B1 | 02-09-2003 |
| | | | US | 5935075 A | 10-08-1999 |
| | | | AU | 2002307207 A1 | 24-07-2003 |
| | | | WO | 03057040 A1 | 17-07-2003 |
| | | | US | 2003171691 A1 | 11-09-2003 |
| | | | US | 2003004430 A1 | 02-01-2003 |
| | | | US | 6475159 B1 | 05-11-2002 |
| | | | US | 2004111016 A1 | 10-06-2004 |
| | | | AT | 221338 T | 15-08-2002 |
| | | | AU | 709432 B2 | 26-08-1999 |
| | | | AU | 7368996 A | 09-04-1997 |
| | | | CA | 2231425 A1 | 27-03-1997 |
| | | | DE | 69622764 D1 | 05-09-2002 |
| | | | DE | 69622764 T2 | 24-04-2003 |
| | | | EP | 0955883 A1 | 17-11-1999 |
| | | | JP | 2000511786 T | 12-09-2000 |
| | | | WO | 9710748 A1 | 27-03-1997 |
| JP 03162637 | A | 12-07-1991 | NONE | | |
| EP 1267736 | A | 02-01-2003 | EP | 1267736 A1 | 02-01-2003 |
| | | | DK | 1267736 T3 | 14-03-2005 |
| | | | EP | 1504725 A1 | 09-02-2005 |
| | | | AT | 281121 T | 15-11-2004 |
| | | | AU | 5630301 A | 15-10-2001 |
| | | | CA | 2406200 A1 | 11-10-2001 |
| | | | DE | 60106887 D1 | 09-12-2004 |
| | | | WO | 0174263 A1 | 11-10-2001 |
| | | | ES | 2231484 T3 | 16-05-2005 |
| | | | JP | 2003528687 T | 30-09-2003 |
| | | | US | 2002198465 A1 | 26-12-2002 |
| | | | US | 2003120171 A1 | 26-06-2003 |
| US 6640130 | B1 | 28-10-2003 | US | 2004236229 A1 | 25-11-2004 |
| | | | AU | 5783900 A | 22-01-2001 |
| | | | CA | 2374040 A1 | 11-01-2001 |
| | | | EP | 1196081 A2 | 17-04-2002 |
| | | | JP | 2003503135 T | 28-01-2003 |
| | | | WO | 0101854 A2 | 11-01-2001 |
| US 2003011622 | A1 | 16-01-2003 | EP | 1417648 A2 | 12-05-2004 |
| | | | EP | 1417647 A2 | 12-05-2004 |
| | | | WO | 03007486 A2 | 23-01-2003 |
| | | | WO | 03007487 A2 | 23-01-2003 |
| | | | US | 2004174361 A1 | 09-09-2004 |

EPO FORM P0459

For more details about this annex : see Official Journal of the European Patent Office, No. 12/82